# DEMANDE DE BREVET EUROPEEN

(11) **EP 2 340 807 A2**
(43) Date de publication de la demande: **06.07.2011**
(21) Numéro de dépôt: 10195734.8
(22) Date de dépôt: 17.12.2010
(51) Int. Cl.: A61K 8/31, A61Q 5/08, A61Q 5/10

(54) **Agent de coloration et/ou de décoloration des fibres kératiniques comprenant une composition comprenant un agent alcalinisant et une composition anhydre comprenant un oxydant, l'une ou l'autre des compositions contenant un corps gras**

(30) Priorité: 22.12.2009 FR 0959388
(71) Demandeur: L'Oréal, 75008 Paris (FR)
(72) Inventeur: Deconinck, Gautier, 95210, Saint Gratien (FR); Goget, Caroline, 75018, Paris (FR); Cotteret, Jean, 78600, Maisons Laffite (FR)
(74) Mandataire: Fevrier, Murielle Françoise E.

(57) **Abrégé**

La présente invention a pour objet un agent de coloration et/ou de décoloration des fibres kératiniques, comprenant (a) une composition (A) contenant un ou plusieurs agens alcalinisants, et (b) une composition anhydre (B) comprenant du peroxyde d'hydrogène et/ou un ou plusieurs générateurs de peroxyde d'hydrogène, la composition (A) et/ou la composition (B) comprenant un ou plusieurs corps gras exempts de fonction acide carboxylique et la quantité totale de corps gras exempt de fonction acide carboxylique après mélange de la composition (A) et de la composition (B) étant supérieure à 20 % en poids.

Elle concerne également un procédé de traitement des fibres kératiniques humaines à partir de cet agent de coloration et/ou de décoloration.

L'invention a de même pour objet un dispositif à deux compartiments comprenant dans l'un des compartiments une composition (A) contenant au moins 25 % d'un ou plusieurs corps gras ne contenant pas de fonction acide carboxylique et un agent alcalin et dans l'autre, une composition anhydre (B) comprenant du peroxyde d'hydrogène ou un générateur de peroxyde d'hydrogène.

## Description

La présente invention concerne un agent en deux parties ou plus, destiné à la coloration et/ou la décoloration des fibres kératiniques, en particulier des fibres kératiniques humaines telles que les cheveux.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, habituellement une ou plusieurs bases d'oxydation éventuellement associées à un ou plusieurs coupleurs.

En général, les bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

On connaît également la coloration directe ou semi-permanente. Le procédé classiquement utilisé en coloration directe consiste à appliquer sur les fibres kératiniques des colorants directs qui sont des molécules colorées et colorantes, ayant une affinité pour les fibres, à laisser pauser, puis à les rincer.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques.

Ce type de procédé ne nécessite pas l'emploi d'un agent oxydant pour développer la coloration. Cependant, il n'est pas exclu d'en mettre en oeuvre afin d'obtenir avec la coloration, un effet d'éclaircissement. On parle alors de coloration directe ou semi-permanente en conditions éclaircissantes.

Les procédés de coloration permanente ou encore semi-permanente en conditions éclaircissantes, consistent donc à employer avec la composition tinctoriale, une composition aqueuse comprenant au moins un agent oxydant, en condition de pH alcalin dans la grande majorité des cas. Cet agent oxydant a entre autre pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

Afin d'améliorer les performances des procédés de coloration et/ou de décoloration des fibres kératiniques humaines, et de limiter les désagréments liés à l'emploi d'agents alcalins et d'agents oxydants, il a été proposé d'employer dans les compositions tinctoriales une quantité substantielle d'un ou plusieurs corps gras tels que notamment des huiles.

Cependant, l'incorporation dans ces compositions d'une quantité suffisante d'huile s'avère problématique en terme de stabilité. En effet, ces compositions sont thermodynamiquement instables, sensibles à la température, et leur viscosité évolue dans le temps, d'autant plus lorsque l'on cherche à en augmenter la teneur en huile.

Il existe un besoin d'avoir une efficacité satisfaisante des produits d'éclaircissement et de coloration, notamment en terme de pouvoir éclaircissant ou d'intensité de coloration et/ou de sélectivité, tout en diminuant les effets néfastes liés à la présence simultanée d'agents alcalins et d'agents oxydants tels que le peroxyde d'hydrogène, notamment en diminuant la dégradation des fibres kératiniques et en réduisant les désagréments liés à l'odeur des agents alcalins mis en oeuvre comme l'ammoniaque et les amines.

On cherche donc à augmenter les effets des agents alcalins et/ou des agents oxydants tout en ayant une efficacité tinctoriale ou éclaircissante maximale sur les fibres kératiniques.

Ce but et d'autres sont atteints par la présente invention qui a donc pour objet un agent de décoloration/coloration des cheveux comprenant (a) une composition (A) contenant un ou plusieurs agents alcalinisants et (b) une composition anhydre (B) comprenant du peroxyde d'hydrogène et/ou un ou plusieurs générateurs de peroxyde d'hydrogène; la composition (A) et/ou la composition (B) comprenant un ou plusieurs corps gras exempts de fonction acide carboxylique et la quantité totale de corps gras exempts de fonction acide carboxylique dans l'agent après mélange de l'émulsion directe (A) et de l'émulsion inverse (B) étant supérieure à 20 % en poids.

Elle concerne également un procédé de traitement des fibres kératiniques humaines à partir de cet agent de coloration/décoloration.

L'invention a de même pour objet un dispositif à deux compartiments comprenant dans l'un des compartiments une composition (A) aqueuse contenant un ou plusieurs agents alcalinisants et dans l'autre, une composition anhydre (B) comprenant du peroxyde d'hydrogène ou un générateur de peroxyde d'hydrogène, la composition (A) et/ou la composition (B) comprenant un ou plusieurs corps gras exempts de fonction acide carboxylique et la quantité totale de corps gras exempt de fonction acide carboxylique après mélange de la composition (A) et de la composition (B) étant supérieure à 20 % en poids. ?

.Lorsque l'agent selon l'invention est destiné à la coloration, des fibres kératiniques, la composition (A) comprend en outre un ou plusieurs colorants d'oxydation et/ou un ou plusieurs colorants directs.

A l'inverse, lorsque l'agent selon l'invention est destiné à la seule décoloration des fibres kératiniques, les compositions (A) et (B) ne comprennent pas de colorant direct ni de colorant d'oxydation (bases et coupleurs) ou bien, s'ils sont présents, leur teneur totale ne dépasse pas 0,005% en poids par rapport au poids de chaque composition. En effet, à une telle teneur, seule la composition serait teintée, c'est-à-dire qu'on n'observerait pas d'effet de coloration des fibres kératiniques.

Les compositions (A) et (B) de l'agent de coloration et/ou de décoloration selon la présente invention sont stables, et leur viscosité n'évolue pas ou peu au cours du temps. Il présente ainsi une facilité d'application et une très bonne efficacité, notamment en termes de qualité et d'homogénéité de la coloration et/ou de la décoloration.

En outre, lorsqu'il est destiné à la coloration des fibres kératiniques, l'agent selon l'invention est particulièrement efficace notamment en ce qui concerne la montée des colorants sur les fibres, la puissance et la chromaticité de la coloration obtenue, ainsi que sur le plan de la sélectivité de la coloration le long d'une même fibre, ou entre des fibres différemment sensibilisées.

Lorsqu'il est destiné à la décoloration des fibres kératiniques, l'agent selon l'invention présente des performances éclaircissantes équivalentes voire supérieures à celles obtenues avec les compositions existantes, notamment avec celles à base d'hydroxyde d'ammonium.

L'agent selon l'invention présente également l'avantage de limiter les odeurs agressives lors de sa préparation, ou de son application sur les fibres.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Selon la présente invention, la composition (A) comprend un ou plusieurs agents alcalinisants. Ce ou ces agents alcalinisants peuvent être choisis parmi les agents alcalins minéraux ou organiques ou hybrides ou leurs mélanges.

Le ou les agents alcalins minéraux sont de préférence choisis parmi l'ammoniaque, les carbonates ou bicarbonates alcalins comme les carbonates de sodium ou de potassium et les bicarbonates de sodium ou de potassium, les hydroxydes de sodium ou de potassium ou leurs mélanges.

Le ou les agents alcalins organiques sont de préférence choisis parmi les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

A titre de composés hybrides on peut mentionner les sels des amines citées précédemment avec des acides comme l'acide carbonique, l'acide chlorhydrique.

Le ou les agents alcalins organiques sont par exemple choisis parmi les alcanolamines, les éthylènediamines oxyéthylénées et/ou oxypropylénées, les acides aminés et les composés de formule (1) suivante : 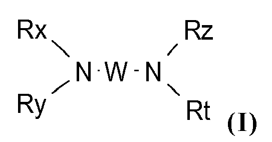 dans laquelle W est un reste alkylène en C₁-C₆ éventuellement substitué par un groupement hydroxyle ou un radical alkyle en C₁-C₆ Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

Par alcanolamine on entend une amine organique comprenant une fonction amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les alcanolamines telles que les mono-, di- ou tri- alcanolamines, comprenant un à trois radicaux hydroxyalkyle, identiques ou non, en C₁-C₄.

A titre d'exemple, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (II) suivante : 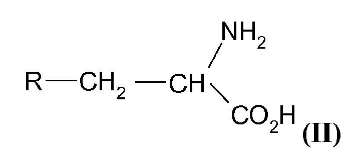 où R désigne un groupe choisi parmi :

| | |
|---|---|
| 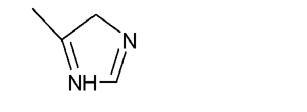 | 3(CH₂)₃N _{H}2 |
| -(CH₂)₂NH₂ | -(CH₂)₂NHCONH₂ |
| 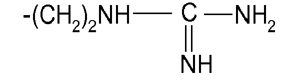 | |

Les composés correspondants à la formule (II) sont l'histidine, la lysine, l'arginine, l'ornithine, la citrulline.

L'amine organique peut être aussi choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole .

L'amine organique peut être aussi choisie parmi les dipeptides d'acides aminés. A titre de dipeptides d'acides aminés utilisables dans la présente invention, on peut notamment citer la carnosine, l'anserine et la baleine

L'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

A titre de composés hybrides on peut mentionner en particulier utiliser le carbonate de guanidine ou le chlorhydrate de monoéthanolamine.

Le ou les agents alcalinisants sont de préférence choisis parmi l'ammoniaque, les carbonates alcalins, les alcanolamines ainsi que leurs dérivés, les hydroxydes de sodium ou de potassium, et les composés de formule (I)

Selon un mode de réalisation particulier, la composition (A) contient en tant qu'agents alcalins au moins une amine organique, de préférence au moins une alcanolamine. Lorsque la composition contient plusieurs agents alcalins dont une alcanolamine et de l'ammoniaque ou un de ses sels, la ou les amines organiques sont de préférence majoritaire en poids par rapport à la quantité ammoniac.

Selon un mode de réalisation préféré de la présente invention, lorsque la composition (A) contient de l'ammoniaque, elle contient également une ou plusieurs alcanolamines, et la quantité pondérale d'alcanolamine(s) dans la composition (A) est supérieure à la quantité pondérale d'ammoniac dans cette même composition.

Selon un autre mode de réalisation préféré de la présente invention, la composition (A) ne contient pas d'ammoniaque.

Généralement, la composition (A) présente une teneur en agents alcalinisant(s) allant de 0,1 à 40% en poids, de préférence de 0,5 à 20% en poids, par rapport au poids de cette composition.

De préférence, la composition (A) présente un pH supérieur ou égal à 8, et plus préférentiellement un pH allant de 8,5 à 11,5.

Ce pH peut également être ajusté à la valeur souhaitée par l'emploi, en plus du ou des agents alcalinisants, d'un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

La composition (A) comprend de préférence au moins 25 % de corps gras ne contenant pas de fonction acide carboxylique, de préférence au moins 30%, mieux au moins 35% en poids.

Par corps gras, on entend dans la présente invention, un composé organique insoluble dans l'eau, à température ordinaire (25°C) et à pression atmosphérique (760 mm de Hg), c'est-à-dire qui présente une solubilité en poids dans l'eau inférieure à 5% et de préférence à 1% encore plus préférentiellement à 0,1%. Les corps gras présentent dans leur structure au moins un enchaînement d'au moins deux groupements siloxanes ou une chaîne hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène, l'huile de vaseline ou le décaméthylcyclopentasiloxane.

Dans le cadre de l'invention, les corps gras ne comprennent pas de fonction acide carboxylique. Par « corps gras ne contenant pas de fonction acide carboxylique », on désigne un corps gras ne contenant pas de groupement -COOH, ni de groupement -COO-.

Selon l'invention, les corps gras ne contenant pas de fonction acide carboxylique sont choisis parmi les composés liquides ou pâteux à température ambiante et à pression atmosphérique, de préférence liquide.

Le ou les corps gras ne contenant pas de fonction acide carboxylique sont notamment choisis parmi les alcanes inférieurs en C₆-C₁₆, les huiles non siliconées d'origine minérale, végétale, animale ou synthétique, les alcools gras, les esters d'acide gras, les esters d'alcool gras, les cires non siliconées et les silicones.

II est précisé qu'au sens de l'invention, les alcools gras et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs en C₆-C₁₆, ces derniers sont linéaires ou ramifiés, éventuellement cycliques. A titre d'exemple, on peut citer l'hexane et le dodécane, les isoparaffines comme l'isohexadécane et l'isodécane.

Comme huiles d'origine minérale, végétale, animale ou synthétique utilisables dans la présente invention, on peut citer par exemple :
- les huiles hydrocarbonées d'origine animale, telles que le perhydrosqualène ;
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
   - les hydrocarbures linéaires ou ramifiés de plus de 16 atomes de carbone, d'origine minérale ou synthétique, tels que les huiles de paraffine, la vaseline, l'huile de vaseline, les polydécènes, les polyisobutènes hydrogénés tels que Parléam®.
   - les huiles fluorées comme le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

En ce qui concerne les esters d'acides gras et/ou d'alcools gras, ils sont avantageusement différents des triglycérides mentionnés ci-dessus ; on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total d'atomes de carbone des esters étant plus particulièrement supérieur ou égal à 10.

Parmi les monoesters , on peut citer le béhénate de dihydroabiétyle ; le béhénate d'octyldodécyle ; le béhénate d'isocétyle; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-hexyle, le palmitate de 2-octyldécyle, les myristates d'alkyles tels que le myristate d'isopropyle, de butyle, de cétyle, de 2-octyldodécyle, de myristyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; le trilactate de glycéryle ; le trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. II est rappelé que l'on entend par «sucre», des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou dioléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48 % de di-, tri-, et tétra-ester, de 45% de monoester et 55% de di-, tri-, et tétra-ester, de 39% de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20% de monoester et 80 % de di-triester-polyester;
- le mono-di-palmito-stéarate de sucrose commercialisé par la société Goldschmidt sous la dénomination Tegosoft® PSE.

La cire ou les cires non siliconées susceptibles d'être utilisées comme corps gras sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones utilisables comme corps gras sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. II s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.
   On peut également citer les cyclocopolymères du type diméthylsiloxanes/ méthylalkylsiloxane, tel que la SILICONE VOLATILE® FZ 3109 commercialisée par la société UNION CARBIDE, de formule : 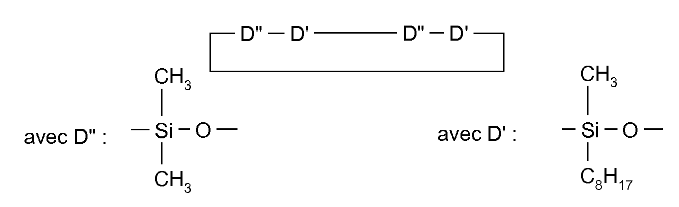 On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10-6m²/s à 25° C. II s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm2/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de diméthiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10-6m²/S. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

R₂SiO_{2/2}, R₃SiO₁/₂, RSiO₃/₂ et SiO₄/₂

dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre les silicones décrites ci-dessus, les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10-5 à 5.10-2m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant:
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, le ou les corps gras ne comprennent pas de motifs oxyalkylénés, ni de motifs glycérolés.

Dans une variante de l'invention, le ou les corps gras sont liquides à température ambiante (25°C) et à pression atmosphérique (760 mmHg).

Le ou les corps gras ne contenant pas de fonction acide carboxylique sont de préférence choisis parmi les alcanes inférieurs en C₆-C₁₆, les huiles non siliconées d'origine minérale, végétale ou synthétique, les alcools gras, les esters d'acide gras, les esters d'alcool gras, les silicones.

De préférence, ledit ou lesdits corps gras de la composition selon l'invention sont non siliconés.

Selon un mode de réalisation préféré, ledit ou lesdits corps gras est ou sont choisis parmi les huiles de paraffine, l'huile de vaseline, les polydécènes, les esters d'acides gras et/ou d'alcools gras liquides, et leurs mélanges.

Selon un mode de réalisation particulièrement préféré, le corps gras est l'huile de vaseline.

Selon une variante, l'émulsion directe utile dans la présente invention comprend au moins un colorant d'oxydation et/ou un colorant direct.

Les colorants d'oxydation sont en général choisis parmi les bases d'oxydation et les coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1026978 et GB 1153196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2733749 et DE 19543 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1 H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1 H,5H-pyrazolo[1,2-a]pyrazol-1 -one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1 -yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préfère utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

Parmi les coupleurs pouvant être utilisés, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

Les sels d'addition des bases d'oxydation et des coupleurs sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est(sont) présent(s), représente chacun avantageusement de 0,0001 à 10 % en poids par rapport au poids total de la composition, et de préférence de 0,005 à 5 % en poids par rapport au poids total de la composition.

Les colorants directs pouvant être utilisés sont par exemple les colorants synthétiques ou naturels, choisis parmi les espèces ioniques ou non ioniques, de préférence cationiques ou non ioniques.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges. En particulier, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Parmi les colorants directs benzéniques utilisables selon l'invention, on peut citer de manière non limitative les composés suivants :
- 1,4-diamino-2-nitrobenzène,
- 1-amino-2 nitro-4-ß- hydroxyéthylaminobenzène
- 1-amino-2 nitro-4-bis(β-hydroxyéthyl)-aminobenzène
- 1,4-bis(ß -hydroxyéthylamino)-2-nitrobenzène
- 1-ß-hydroxyéthylamino-2-nitro-4-bis-(β-hydroxyéthylamino)-benzène
- 1-ß-hydroxyéthylamino-2-nitro-4-aminobenzène
- 1-β-hydroxyéthylamino-2-nitro-4-(éthyl)(ß-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropytamino-4-triftuorométhyt-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (I) à (IV), et de préférence les composés de formules (III) , (IV), (V) et (V') : 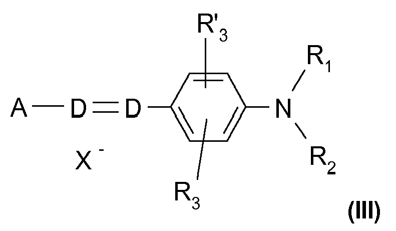 dans laquelle :
D représente un atome d'azote ou le groupement -CH, de préférence un atome d'azote,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X - représente un anion de préférence choisi parmi le chlorure, le méthyl sulfate et l'acétate,
A représente un groupement choisi par les structures A₁ à A₁₈, et de préférence A₁, A₄, A₇, A₁₃ et A₁₈, suivantes : 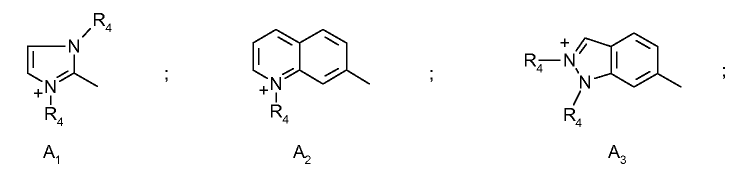 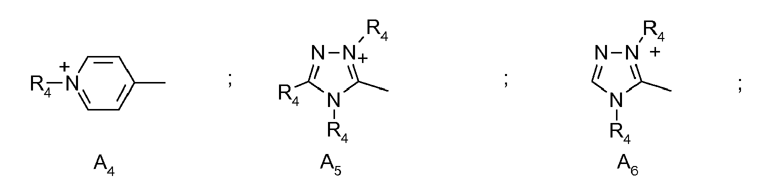 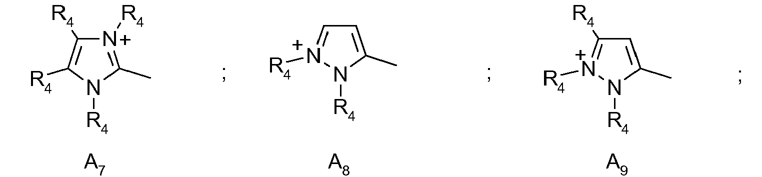 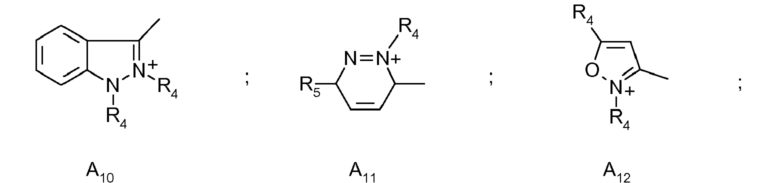 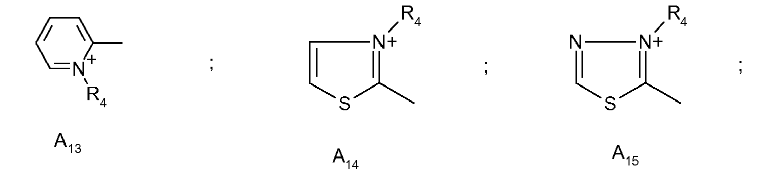 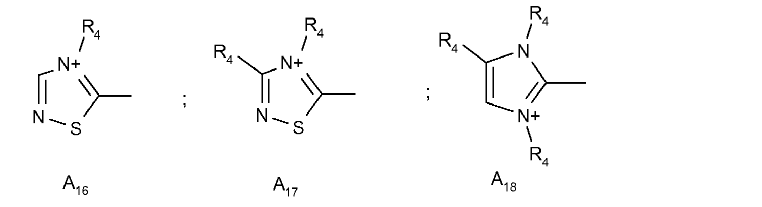 dans lesquelles R₄ représente un radical alkyle en C₁-C₄ pouvant être substitué par un radical hydroxyle et R₅ représente un radical alcoxy en C₁-C₄ ; 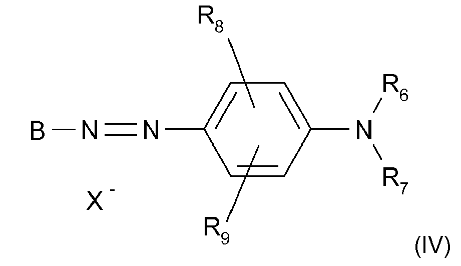 dans laquelle :
   R₆ représente un atome d'hydrogène ou un radical alkyle en C₁-C₄,
   R₇ représente un atome d'hydrogène, un radical alkyle pouvant être substitué par un radical - CN ou par un groupement amino, un radical 4'-aminophényle ou forme avec R₆ un hétérocycle éventuellement oxygéné et/ou azoté pouvant être substitué par un radical alkyle en C₁-C₄,
   R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   X - représente un anion de préférence choisi parmi le chlorure, le méthyl sulfate et l'acétate, B représente un groupement choisi par les structures B1 à B6 suivantes : 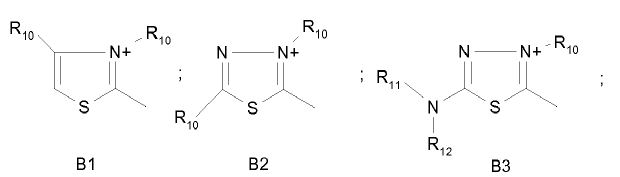 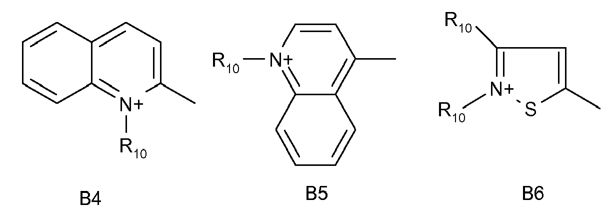 dans lesquelles R₁₀ représente un radical alkyle en C₁-C₄, R₁₁ et R₁₂, identiques ou différents, représentent un atome d'hydrogène ou un radical alkyle en C₁-C₄ ; 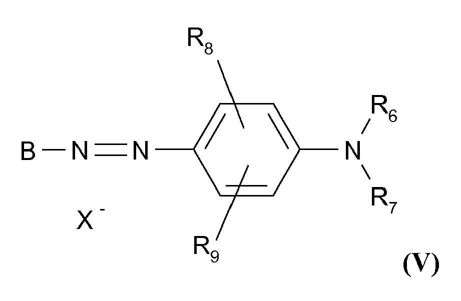 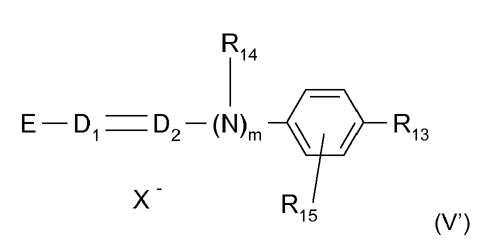 dans lesquelles :
      R₁₃ représente un atome d'hydrogène, un radical alcoxy en C₁-C₄, un atome d'halogène tel que le brome, le chlore, l'iode ou le fluor,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C_{4,}
      D₁ et D₂, identiques ou différents, représentent un atome d'azote ou le groupement -CH,
      m=0 ou 1,
   étant entendu que lorsque R₁₃ représente un groupement amino non substitué, alors D₁ et D₂ représentent simultanément un groupement -CH et m = 0,
   X - représente un anion de préférence choisi parmi le chlorure, le méthyl sulfate et l'acétate,
   E représente un groupement choisi par les structures E1 à E8, et de préférence E1, E2 et E7, suivantes : 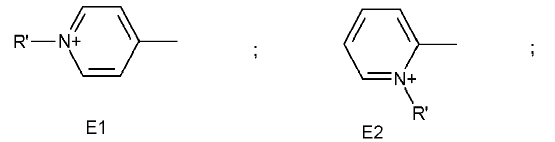 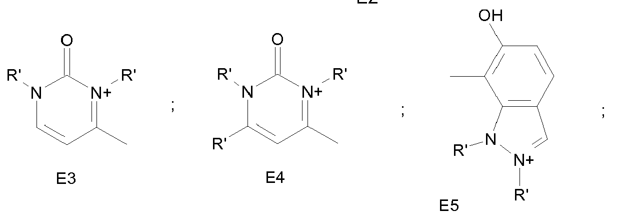 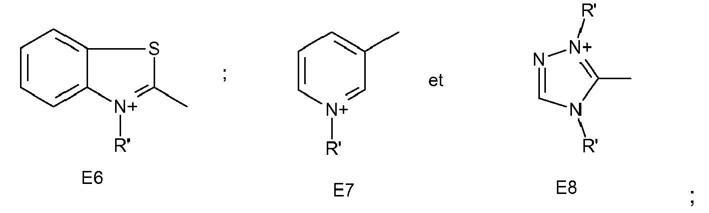 dans lesquelles R' représente un radical alkyle en C₁-C₄;
      lorsque m = 0 et que D₁ représente un atome d'azote, alors E peut également désigner un groupement de structure E9 suivante : 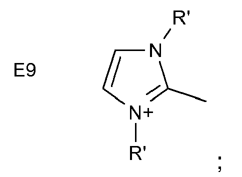 dans laquelle R' représente un radical alkyle en C₁-C₄.

      G—N=N—J (VI)

      dans laquelle :
      le symbole G représente un groupement choisi parmi les structures G₁ à G₃ suivantes : 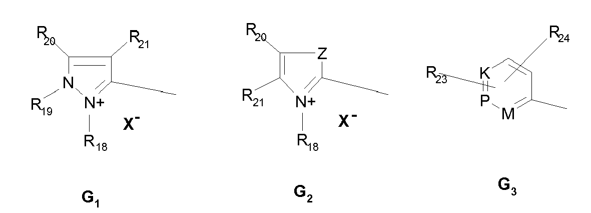
         structures G₁ à G₃ dans lesquelles,
         R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
         R₁₉ désigne un radical alkyle en C₁-C₄ ou un radical phényle;
         R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂;
         R₂₀ peut désigner en outre un atome d'hydrogène;
         Z désigne un atome d'oxygène, de soufre ou un groupement -NR₁₉;
         M représente un groupement -CH, -CR (R désignant alkyle en C₁-C₄),
         ou -NR₂₂(X⁻)r;
         K représente un groupement -CH, -CR (R désignant alkyle en C₁-C₄),
         Ou -NR₂₂(X⁻)r;
         P représente un groupement -CH, -CR (R désignant alkyle en C₁-C₄),
         ou -NR₂₂(X-)r; r désigne zéro ou 1;
         R₂₂ représente un atome 0⁻, un radical alcoxy en C₁-C₄, ou un radical alkyle en C₁-C₄;
         R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
         X⁻ représente un anion de préférence choisi parmi le chlorure, l'iodure, le méthyl sulfate, l'éthyl sulfate, l'acétate et le perchlorate;
         sous réserve que,
         si R₂₂ désigne O‾, alors r désigne zéro;
         si K ou P ou M désignent -N-alkyle C₁-C₄ X‾, alors R₂₃ ou R₂₄ est ou non différent d'un atome d'hydrogène;
         si K désigne -NR₂₂(X‾)ᵣ , alors M= P= -CH, -CR;
         si M désigne -NR₂₂(X‾)ᵣ , alors K= P= -CH, -CR;
         si P désigne -NR₂₂(X‾)ᵣ , alors K= M et désignent -CH ou -CR;
         si Z désigne un atome de soufre avec R₂₁ désignant alkyle en C₁-C₄ , alors R₂₀ est différent d'un atome d'hydrogène;
         si Z désigne -NR₂₂ avec R₁₉ désignant alkyle en C₁-C₄, alors au moins l'un des radicaux R₁₈, R₂₀ ou R₂₁ du groupement de structure G₂ est différent d'un radical alkyle en C₁-C₄;
         le symbole J représente :
            - (a) un groupement de structure J₁ suivante : 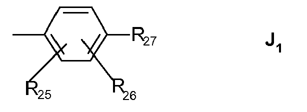 structure J₁ dans laquelle,
               R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, - NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
               R₂₆ représente un atome d'hydrogène, un atome d'halogène choisi parmi le chlore, le brome, l'iode et le fluor, un radical alkyle en C₁-C₄, alcoxy en C₁-C₄,
               ou forme avec R₂₇ ou R₂₈ un cycle à 5 ou 6 chaînons contenant ou non un ou plusieurs hétéroatomes choisis parmi l'azote, l'oxygène ou le soufre;
               R₂₇ représente un atome d'hydrogène, un radical -OH, un radical -NHR₂₈, un radical - NR₂₉ R₃₀;
               R₂₈ représente un atome d'hydrogène, un radical alkyle en C₁-C₄, un radical monohydroxyalkyle en C₁-C₄, polyhydroxyalkyle en C₂-C₄, un radical phényle;
               R₂₉ et R₃₀, identiques ou différents, représentent un radical alkyle en C₁-C₄ , un radical monohydroxyalkyle en C₁-C₄, polyhydroxyalkyle en C₂-C₄;
            - (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
               et notamment un groupement de structure J₂ suivante : 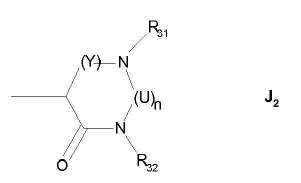 structure J₂ dans laquelle,
               R₃₁ et R₃₂, identiques ou différents, représentent un atome d'hydrogène, un radical alkyle en C₁-C₄, un radical phényle;
               Y désigne le radical -CO- ou le radical 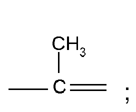
               n = 0 ou 1, avec, lorsque n désigne 1, U désigne le radical -CO- .
Dans les structures (III) à (VI) définies ci-dessus le groupement alkyle ou alcoxy en C₁-C₄ désigne de préférence méthyle, éthyle, butyle, méthoxy, éthoxy.

Parmi les composés de formules (III) et (V), on préfère les composés suivants :

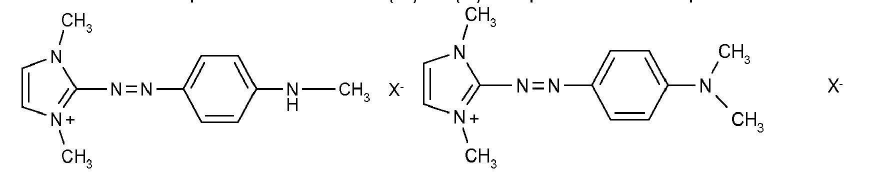

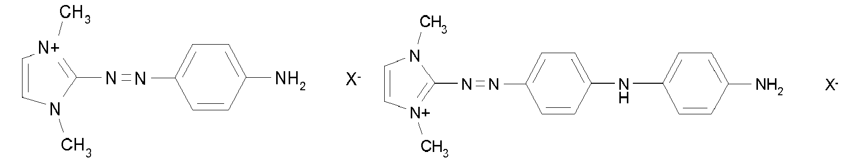

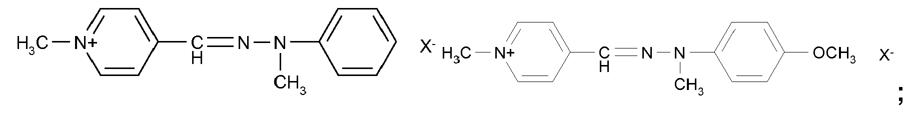

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,y-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-p-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous :

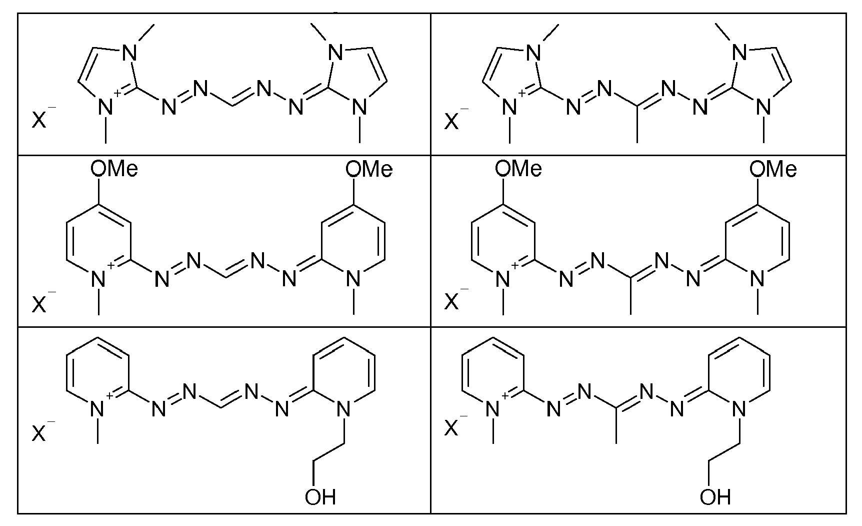

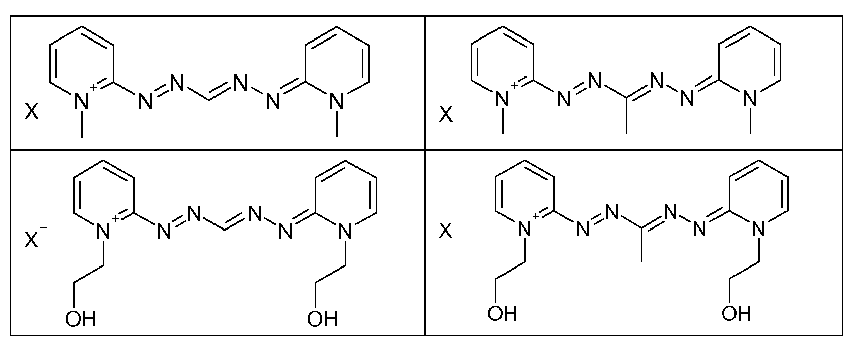

X‾ représente un anion de préférence choisi parmi le chlorure, l'iodure, le méthyl sulfate, l'éthyl sulfate, l'acétate et le perchlorate.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, l'hématoxyline, l'hémateine, la brasiline, la brasiléine, les orcéines. On peut également utiliser les extraits ou décoctions contenant ces colorants naturels et notamment les cataplasmes ou extraits à base de henné.

Les colorants directs peuvent être des colorants monochromophoriques (c'est-à-dire ne comprenant qu'un seul colorant) ou polychromophoriques, de préférence di- ou tri-chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement interrompue par au moins un groupement ammonium quaternaire substitué par deux groupements alkyle en C₁-C₁₅ éventuellement substitués ; le bras de liaison ne comprenant pas de groupement nitro, nitroso ou peroxo.

Si les hétérocycles ou noyaux aromatiques (phényle ou naphtyle) sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle; un radical alcoxy en C₁-C₂ ; un radical hydroxyalcoxy en C₂-C₄ ; un radical amino ; un radical amino substitué par un ou deux radicaux alkyle, identiques ou différents, en C₁-C₄ éventuellement porteurs d'au moins un groupement hydroxyle.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en C₁-C₆, un noyau phényle éventuellement substitué, ou d'au moins un groupement N(R')₂ avec R' identiques ou non, représentant un atome d'hydrogène, un radical alkyle éventuellement substitué en C₁-C₆, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. II est à noter de même que l'hétérocycle peut éventuellement comprendre un autre hétéroatome différent de l'azote, comme le soufre ou l'oxygène.

La liaison entre le bras de liaison, tel que défini précédemment, et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Lorsqu'ils sont présents, le ou les colorants directs représentent plus particulièrement de 0,0001 à 10% en poids du poids total de la composition, et de préférence de 0,005 à 5 % en poids.

La composition (A) peut contenir un ou plusieurs tensioactifs. Les tensio-actifs utiles peuvent être anioniques, cationiques, amphotères ou non ioniques.

Le ou les tensioactifs utilisables sont de préférence choisis parmi les tensioactifs non ioniques et les tensioactifs anioniques, et de préférence parmi les tensioactifs non ioniques.

Les tensioactifs anioniques sont plus spécialement choisis parmi les sels (en particulier sels de métaux alcalins, notamment de sodium, sels d'ammonium, sels d'amines tels que les sels d'aminoalcools ou sels de métaux alcalino-terreux comme le magnésium) des composés suivants :
- les alkylsulfates, les alkyléthersulfates, alkylamidoéthersulfates, alkylarylpolyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

II est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, et les tensioactifs non ioniques mono- ou polyglycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C_{30,} saturés ou non, linéaires ou ramifiés, oxyalkylénés,
- les amides, en C₈₋C_{30,} saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène,
- et leurs mélanges.

Ces tensioactifs présentent un nombre de moles d'oxyde d'éthylène et/ou de propylène compris entre 1 et 100, de préférence entre 2 et 50. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀ oxyéthylénés, et les esters d'acides en C₈-C₃₀ et de polyéthylèneglycols.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]m-H

dans laquelle R représente un radical alkyle ou alcényle, linéaire ou ramifié, en C₈-C₄₀, de préférence en C₈-C_{30,} et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

Selon un mode de réalisation préféré de la présente invention, la composition (A) comprend un ou plusieurs tensio-actifs non ioniques.

Dans une variante de ce mode de réalisation la composition (A) contient un ou plusieurs tensioactifs non ioniques de HLB supérieure ou égale à 8.

Lorsqu'il est ou sont présents, le ou les tensio-actifs représentent une quantité variant de 1 à 20% en poids de tensioactif(s), plus préférentiellement de 1 à 15% en poids, et mieux de 2 à 10% en poids, par rapport au poids total de la composition (A).

La composition (A) utile dans l'invention peut comprendre une ou plusieurs silices pyrogénées.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

Lorsqu'elle est présente, la silice pyrogénée représente de 1 à 30 % en poids par rapport au poids de la composition

La composition (A) utile dans la présente invention peut également comprendre un ou plusieurs agents épaississants.

Ces agents épaississants peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose) ou la gomme de guar et ses dérivés (hydroxypropylguar), les argiles et notamment les bentonites et les hectorites et leurs dérivés

La teneur en agent(s) épaississant(s) s'ils sont présents, varie habituellement de 0,01% à 20% en poids, par rapport au poids de la composition (A), de préférence de 0,1 à 5% en poids.

La composition (A) comprend l'eau et éventuellement un ou plusieurs solvants organiques.

A titre de solvant organique, on peut par exemple citer les monoalcools linéaires ou ramifiés, de préférence saturés, comprenant 2 à 6 atomes de carbone, tels que l'alcool éthylique, l'alcool isopropylique,; les alcools aromatiques tels que l'alcool benzylique, l'alcool phényléthylique ; ; les polyols à plus de deux fonctions hydroxyles tels que le glycérol ; les éthers de polyol tels que, par exemple, les éthers monométhylique, monoéthylique et monobutylique d'éthylèneglycol, le propylèneglycol ou ses éthers tels que, par exemple, le monométhyléther de propylèneglycol,; ainsi que les alkyléthers de diéthylèneglycol, notamment en C₁-C₄, comme par exemple, le monoéthyléther ou le monobutyléther du diéthylèneglycol, seuls ou en mélange.

Les solvants organiques, quand ils sont présents, représentent généralement entre 1 et 40 % en poids par rapport au poids total de chacune des compositions, et de préférence entre 5 et 30 % en poids par rapport au poids total la composition (A)

La quantité d'eau dans la composition (A) peut varier de 5 à 90% d'eau, mieux de 10 à 80% en poids .

La phase aqueuse de la composition (A) qui comprend l'eau et les composés solubles dans l'eau à température ambiante (25°C) et à pression atmosphérique (760 mm Hg) représente de préférence de 10 à 85% en poids de la composition (A)

La composition (A) peut se présenter sous des formes diverses, telles que sous forme de liquides, de laits , de crèmes, ou sous toute autre forme appropriée pour réaliser une teinture des fibres kératiniques, et notamment des cheveux humains.

De préférence, elle se présente sous la forme d'un lait ou d'une crème.

La composition (A) peut être une émulsion directe ou une émulsion inverse.

De préférence la composition (A) est une émulsion directe.

La composition (B) est une composition anhydre contenant du peroxyde d'hydrogène et/ou un ou plusieurs précurseurs de peroxyde d'hydrogène.

A titre de générateur de peroxyde d'hydrogène utile dans la présente invention, on peut citer les complexes polymériques pouvant libérer du peroxyde d'hydrogène tels que le polyvinylpyrrolidone/H₂O₂ en particulier se présentant sous forme de poudres et les autres complexes polymériques décrits dans US 5,008,093 ; US 3,376,110 ; US 5,183,901.On peut aussi citer le peroxyde d'urée, les perborates et percarbonates de métaux alcalins ou alcalinoterreux. ou d'ammonium.

On peut noter que les persulfates de métaux alcalins ou alcalinoterreux. ou d'ammonium ne sont pas inclus dans ces précurseurs car dans les mécanismes d'oxydoréduction mettant en oeuvre ces persulfates il n'y a pas libération de peroxyde d'hydrogène.

Les compositions anhydres de peroxyde d'hydrogène utilisable dans la présente invention sont connues de la technique. Notamment, le document GB-A-632 084 décrit des solutions liquides antiseptiques de peroxyde d'urée résultant de l'association de peroxyde d'hydrogène et d'urée dans un alcool polyhydrique ; le document DE-A-1 262 982 décrit des solutions de peroxyde d'hydrogène dans des di ou polyesters d'acides dicarboxyliques insaturés ; le document FR-A-2 145 400 décrit des solutions d'un composé peroxydé tel que le peroxyde d'hydrogène dans des esters d'alcoyle ou dans des acides organiques ; le document EP-121 660 décrit un procédé de préparation de solutions de peroxyde d'hydrogène dans divers solvants organiques utilisables pour des réactions d'oxydation ou d'époxydation. Le document EP 193471 décrit une composition anhydre de peroxyde d'hydrogène dans un solvant organique utilisable pour une application cosmétique. Les compositions décrites dans ce document qui contiennent du peroxyde d'hydrogène et un ou plusieurs solvants organiques sont particulièrement préférées.

Les compositions anhydres de peroxyde d'hydrogène et/ou de précurseurs de peroxyde d'hydrogène contiennent de préférence moins de 5% en poids d'eau, mieux moins de 1% d'eau.

La composition(B) peut se présenter sous forme d'une poudre, sous forme d'une pate ou d'une crème ou sous forme d'un liquide épaissi ou non.

La forme poudre est de préférence utilisée pour les précurseurs de peroxyde d'hydrogène. Ces formes poudres peuvent être éventuellement compactées.

Dans les formes poudre, l'agent oxydant peut représenter de 5 à 100% en poids de la composition (B).Les éventuels adjuvants pulvérulents présents dans ces formes poudres peuvent être hydrosolubles ou non. Parmi les adjuvants pulvérulents hydrosolubles, on peut notamment citer les polysaccharides tels que le glucose, le lactose, le saccharose et le sorbitol. Parmi les adjuvants pulvérulents figurent aussi les agents acidifiants ou alcalinisants solides. Parmi les adjuvants pulvérulents non solubles dans l'eau on peut notamment citer les silices mentionnées ci-dessus, les argiles, les polymères tels que les polyamides, le téflon,les polyméthacrylates d'alkyle, les billes de verre.

Dans une variante de l'invention la composition (B) consiste en une dispersion ou une solubilisation du peroxyde d'hydrogène et/ou des précurseurs de peroxyde d'hydrogène dans un solvant ou mélange de solvants.

Ce ou ces solvants organiques peuvent être de nature très variée notamment :
1) les corps gras ne contenant pas de fonction acide carboxylique tels que ceux mentionnés précédemment.
1) les alcools au moins partiellement hydrosolubles (de préférence ayant une solubilité supérieure ou égale à 4% dans l'eau à température ambiante (25°C) et pression atmosphérique (760 mmHg) tels que l'éthanol, le n-propanol, l'isopropanol, et l'alcool benzylique,
2) les polyols acycliques tels que les glycols et plus particulièrement le propylène glycol, le diéthylène glycol, le dipropylène glycol, le tripropylène glycol, le glycérol, et leurs éthers tels que le monométhyléther de propylène glycol, l'éther monoéthylique du diéthylène glycol , l'éther monoéthylique du propylène glycol et
3) les polyéthers oligomères tels que ceux de l'oxyde d'éthylène, de l'oxyde de propylène et leurs éthers, ainsi que les polyéthers oligomères répondant à la formule (VII) suivante : 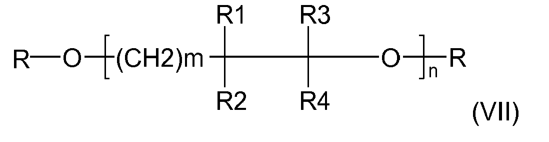 dans laquelle :
   R représente un radical alkyle, linéaire ou ramifié, ayant de 1 à 12 atomes de carbone, R₁, R₂ R₃ et R₄, identiques ou différents, représentent un atome d'hydrogène ou un radical alkyle ayant de 1 à 6 atomes de carbone, au moins deux des radicaux R₁, R₂, R₃ ou R₄ représentant un atome d'hydrogène, m est 1 à 4, et n est une valeur moyenne supérieure ou égale à 2 et de préférence comprise entre 4 et 50, le nombre d'atomes de carbone dans chaque unité répétitive, identique ou différente, étant au moins égal à 4.

La masse moléculaire moyenne en nombre de ces polyéthers oligomères est généralement comprise entre 200 et 5 000 et leur viscosité entre 0,002 et 1 Pa.s mesurée à 25 °C et de préférence entre 0,01 et 0,1 Pa.s.
Certains de ces oligomères sont solubles dans l'eau d'autres non.

Parmi les polyéthers oligomères représentés par la formule ci-dessus, le diméthyléther du polytétrahydrofuranne répondant à la formule (VIII) suivante est particulièrement préféré : 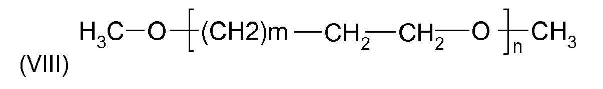 dans laquelle n est une valeur moyenne comprise entre 4 et 10.

Il va de soi que le solvant organique doit être stable en présence du peroxyde d'hydrogène et/ou du ou des précurseurs de peroxyde d'hydrogène dans les conditions d'utilisation de la composition anhydre.

Les procédés de préparation des compositions anhydres de peroxyde d'hydrogène sont connus et ont été abondamment décrits dans la littérature. Pour cette préparation, deux procédés différents peuvent être envisagés selon la nature du solvant organique. Lorsque le solvant organique n'est pas miscible à l'eau, le peroxyde d'hydrogène est extrait, sous forte agitation, à partir d'une solution aqueuse à 60 % d'eau oxygénée (200 volumes). Après agitation entre 3 à 8 heures, on sature la phase aqueuse par du chlorure de sodium et on décante la phase organique qui est ensuite séchée sur sulfate de sodium. Lorsque le solvant organique est miscible à l'eau, l'eau est éliminée par distillation azéotropique.

Dans ce cas, on utilise comme co-solvant susceptible de former un mélange azéotropique avec l'eau, l'acétonitrile, le tertio-butanol, le cyclohexane, l'heptane, le pentane, le dichloro-1,2 éthane ou l'acétate d'éthyle.

L'entraînement azéotropique de l'eau se fait à température d'ébullition du mélange. Après refroidissement, on sèche sur sulfate de sodium anhydre.

Le dosage du peroxyde d'hydrogène dans le solvant organique est ensuite déterminé par polarographie.

En fonction de la nature du solvant, la teneur en peroxyde d'hydrogène des compositions peut varier dans de larges limites.

Comme procédé par distillation azéotropique, on peut en particulier faire référence au document EP 121 660.

Lorsque la composition(B) contient du peroxyde d'hydrogène, sa concentration peut varier de 1 à 50%, de préférence de 4 à 30%, encore plus préférentiellement de 4 à 20% mieux de 5 à 15% en poids par rapport au poids de la composition (B).

La composition anhydre de peroxyde d'hydrogène peut également contenir d'autres ingrédients notamment des produits permettant d'augmenter la viscosité ceci en vue d'éviter tout écoulement de la composition lors de l'application.

Elle peut contenir divers adjuvants utilisés habituellement en cosmétique comme des tensio-actifs, par exemple non ioniques ou anioniques, des épaississants, etc

En particulier, elle peut contenir un ou plusieurs corps gras exempts de fonction acide carboxylique, tels que définis précédemment, soit à titre de solvant(s) pour le peroxyde d'hydrogène et/ou le ou les précurseurs de peroxyde d'hydrogène, soit à titre de composés additionnels.

Comme indiqué précédemment l'agent de coloration/décoloration obtenu à partir de la composition (A) et de la composition anhydre (B) est tel que la quantité de corps gras après mélange des deux compositions est supérieure ou égale à 20% en poids, par rapport au poids total du mélange de ces deux compositions.

Ainsi, le ou les corps gras ne contenant pas de fonction acide carboxylique peuvent être présents dans la composition (A), ou la composition (B), ou les deux compositions à la fois, pourvu que la quantité totale de ces corps gras dans le mélange des compositions (A) et (B) soit au moins égale à 20% en poids, par rapport au poids total du mélange des deux compositions (A) et (B).

De préférence, la quantité totale de corps gras ne contenant pas de fonction acide carboxylique dans le mélange des compositions (A) et (B) représente au moins 25% en poids, et plus préférentiellement au moins 30% en poids, par rapport au poids total dudit mélange.

La quantité totale de corps gras ne contenant pas de fonction acide carboxylique dans le mélange des compositions (A) et (B) est avantageusement inférieure ou égale à 90% en poids, et de préférence inférieure ou égale à 70% en poids, par rapport au poids total dudit mélange.

Dans une variante de l'invention, l'agent de l'invention n'est constitué que par les deux compositions (A) et (B).

La présente invention a également pour objet un procédé de coloration et/ou de décoloration des fibres kératiniques, comprenant l'application sur lesdites fibres de l'agent tel que décrit ci-avant.

Selon l'invention, l'agent appliqué sur les fibres kératiniques résulte du mélange de la composition (A) et de la composition anhydre (B), ce mélange étant réalisé soit avant application sur les fibres kératiniques (préparation extemporanée) soit directement sur les fibres kératiniques (application successive sur les fibres de la composition (A) et de la composition anhydre (B) sans rinçage intermédiaire).

Ainsi, selon une première variante du procédé selon l'invention, on applique sur les fibres kératiniques, sèches ou humides, successivement et sans rinçage intermédiaire, la composition (A) et la composition anhydre (B).

Selon une deuxième variante du procédé selon l'invention, on applique sur les fibres kératiniques, sèches ou humides, une composition obtenue par mélange extemporané, avant l'application, la composition (A) et la composition anhydre (B).

Dans ce cas, alors la durée entre le mélange de la composition (A) et de la composition anhydre (B) et l'application du mélange sur les cheveux n'excède pas de préférence 30 minutes, de préférence 10 minutes, de manière encore préférée 5 minutes.

Indépendamment de la variante mise en oeuvre, le rapport pondéral de la quantité de la composition (A) utilisée à la quantité de la composition anhydre (B) utilisée peut varier de 0,2 à 50 et de préférence de 0,3 à 10, mieux de 0.3 à 1.

En outre, indépendamment de la variante mise en oeuvre, le mélange présent sur les fibres (résultant soit du mélange extemporané de la composition (A) et de la composition anhydre (B) soit de l'application successive de celles-ci) est laissé en place pour une durée, en général, de l'ordre de 1 minute à 1 heure, de préférence de 5 minutes à 30 minutes.

La température durant le procédé est classiquement de préférence comprise entre la température ambiante (entre 15 à 25°C) et 80°C, de préférence entre la température ambiante et 60°C.

A l'issue du traitement, les fibres kératiniques sont éventuellement rincées à l'eau, subissent éventuellement un lavage avec un shampooing suivi d'un rinçage à l'eau, avant d'être séchées ou laissées à sécher.

Enfin, l'invention a également pour objet un dispositif à plusieurs compartiments ou « kit » de coloration et/ou de décoloration, comprenant un premier compartiment renfermant une composition (A), ainsi qu'un deuxième compartiment renfermant une composition anhydre (B), les compositions (A) et (B) étant telles que décrites ci-avant.

Ce dispositif peut également comprendre une ou plusieurs compositions de lavage et/ou de conditionnement des fibres kératiniques, destinés à être appliquées avant et/ou après le traitement de coloration et/ou de décoloration selon l'invention.

Les exemples suivants servent à illustrer l'invention sans toutefois présenter un caractère limitatif.

### EXEMPLES

### EXEMPLE 1

On prépare les compositions suivantes :
L'émulsion directe A1 suivante a été préparée selon un procédé d'inversion de phase en température (procédé PIT)

### Procédé de fabrication:

1- Chauffer la phase A au bain marie sous rayneri (400 tr/min). On obtient une émulsion blanche fluide qui devient translucide vers 68°C (passage par une phase de microémulsion) et s'épaissit au-delà.
2- Dès que l'émulsion s'épaissit, ôter le bain marie : laisser refroidir sous la même agitation.
3- Vers 50°C, introduire le gélifiant (carbopol).
4- A Température ambiante, introduire, l'éthanol, la monoéthanolamine , les colorants , l'acide ascorbique , le chélatant et réajuster l'eau perdue à l'évaporation (<5%).
On obtient ainsi une émulsion gélifiée translucide de taille de gouttes < 1 µm

| **Composition colorante A1** | | |
|---|---|---|
| **Phase** | **Nom** | **g%** |
| **A** | Beheneth-10 | 6,00 |
| | Glycérine | 9,00 |
| | Palmitate d'éthylhexyle | 17,70 |
| | Huile de vaseline | 45,00 |
| | Eau | 16,00 |
| **B** | Polymère carboxyvinylique synthétisé dans le mélange acétate d'éthyle/cyclohexane(CARBOPOL 980) | 0,30 |
| **C** | Ethanol | 2,00 |
| | Monoéthanolamine | 4,00 |
| | Acide diethylene triamine pentacetique | 0.015 |
| | Acide ascorbique | 0,25 |
| | 1-methyl-2,5-diaminobenzene | 0,14 |
| | 1-hydroxy-4-amino-benzene | 0,17 |
| | Résorcinol | 0,08 |
| | 1-methyl-2hydroxy-4-beta-hydroxyethylamino-benzene | 0,21 |
| | 1-methyl-2-hydroxy-4-amino-benzene | 0,23 |

### Compositions oxydantes :

### Composition B1 (poudre)

| Composition oxydante (g%) | B1 |
|---|---|
| Peroxyde d'uréee | 90 |
| Lactose | 10 |

### Composition B2 (liquide)

| Composition oxydante (g%) | B2 |
|---|---|
| Peroxyde d'hydrogène | 15 |
| Diméthyléther du polytétrahydrofuranne | 85 |

Au moment de l'emploi, on mélange la composition A1 à une composition aqueuse (B1) ou (B2) dans les proportions suivantes :
1 partie de composition A1 avec 0.1 partie de composition oxydante B1
1 partie de composition A1 avec 0.25 partie de composition oxydante B2

Les mélanges sont ensuite appliqués sur des mèche de cheveux gris à 90% blancs. Après 30 minutes de pause à température ambiante (25°C), les cheveux sont rincés, lavés avec un shampooing standard, puis séchés. On obtient alors une coloration blond acajou.

## Revendications

1. Agent de coloration et/ou de décoloration des fibres kératiniques, comprenant (a) une composition (A) contenant un ou plusieurs agens alcalinisants, et (b) une composition anhydre (B) comprenant du peroxyde d'hydrogène et/ou un ou plusieurs générateurs de peroxyde d'hydrogène, la composition (A) et/ou la composition (B) comprenant un ou plusieurs corps gras exempts de fonction acide carboxylique et la quantité totale de corps gras exempt de fonction acide carboxylique après mélange de la composition (A) et de la composition (B) étant supérieure à 20% en poids.

2. Agent selon la revendication 1, **caractérisé en ce que** l'agent alcalinisant est choisi parmi l'ammoniaque, les carbonates alcalins, les alcanolamines ainsi que leurs dérivés, les hydroxydes de sodium ou de potassium, et les composés de formule (I) suivante : 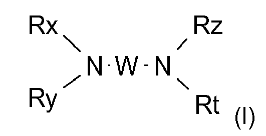 dans laquelle W est un reste alkylène en C₁-C₆ éventuellement substitué par un groupement hydroxyle ou un radical alkyle en C₁-C₆ Rx, Ry, Rz et Rt, identiques ou différents, représentent un atome d'hydrogène, un radical alkyle en C₁-C₆, hydroxyalkyle en C₁-C₆, ou aminoalkyle en C₁-C₆ et est de préférence choisi parmi les alcanolamines.

3. Agent selon l'une quelconque des revendications précédentes, **caractérisé en ce que** la composition (A) contient au moins 25%, de préférence au moins 30% en poids d'une ou plusieurs corps gras ne contenant pas de fonction acide carboxylique, par rapport au poids total de la composition (A).

4. Agent selon l'une quelconque des revendications précédentes, **caractérisé en ce que** le ou les corps gras ne contenant pas de fonction acide carboxylique sont choisis parmi les alcanes inférieurs en C₆-C₁₆, les huiles non siliconées d'origine minérale, végétale, animale ou synthétique, les alcools gras, les esters d'acide gras, les esters d'alcool gras, les cires non siliconées et les silicones.

5. Agent selon l'une quelconque des revendications précédentes, **caractérisé en ce que** le ou les corps gras ne contenant pas de fonction acide carboxylique sont des composés liquides à la température de 25°C et à la pression atmosphérique, et sont de préférence choisis parmi les huiles de paraffine, l'huile de vaseline, les polydécènes, les esters d'acides gras et/ou d'alcools gras liquides, et leurs mélanges, et plus préférentiellement l'huile de vaseline.

6. Agent selon l'une quelconque des revendications précédentes dans lequel la quantité d'eau dans la composition (A) est comprise entre 5% et 90% en, mieux entre 10 et 80% en poids par rapport au poids de la composition (A).

7. Agent selon l'une quelconque des revendications précédentes, **caractérisé en ce que** la composition (A) comprend un ou plusieurs colorants d'oxydation, choisis parmi les bases d'oxydation, éventuellement combinée(s) à un ou plusieurs coupleurs.

8. Agent selon l'une quelconque des revendications précédentes, **caractérisé en ce que** la composition (A) comprend un ou plusieurs colorants directs.

9. Agent selon l'une quelconque des revendications précédentes **caractérisé en ce que** la composition (B) se présente sous forme d'une poudre, d'une pate ou d'une crème ou d'un liquide épaissi ou non.

10. Agent selon l'une quelconque des revendications précédentes **caractérisé en ce que** le ou les précurseurs de peroxyde d'hydrogène sont choisis parmi les complexes polymères/peroxyde d'hydrogène, le peroxyde d'urée, les perborates ou percarbonates de métaux alcalins ou alcalinoterreux.

11. Agent selon l'une quelconque des revendications précédentes caractérisé en ce quelle contient un ou plusieurs solvants organiques et du peroxyde d'hydrogène et/ou un ou plusieurs précurseurs de peroxyde d'hydrogène.

12. Agent selon la revendication précédente dans lequel le ou les solvants organiques de la composition (B) sont choisi parmi les corps gras exempts de fonction acide carboxyliques, les alcools au moins partiellement hydrosolubles, les polyols acycliques, les éthers de polyols acycliques, les éthers du polytétrahydrofuranne

13. Agent selon l'une quelconque des revendications précédentes, **caractérisé en ce que** la quantité totale de corps gras ne contenant pas de fonction acide carboxylique après mélange de la composition (A) et de la composition anhydre (B) représente au moins 25% en poids, et de préférence au moins 30% en poids, par rapport au poids total du mélange de ces deux compositions.

14. Procédé de coloration et/ou de décoloration des fibres kératiniques, comprenant l'application sur lesdites fibres, sèches ou humides, successivement et sans rinçage intermédiaire, de la composition (A) et de la composition anhydre (B) telles que définies dans l'une quelconque des revendications précédentes.

15. Procédé de coloration et/ou de décoloration des fibres kératiniques, comprenant l'application sur lesdites fibres, sèches ou humides, d'une composition obtenue par mélange extemporané de la composition (A) et de la composition anhydre (B) telles que définies dans l'une quelconque des revendications 1 à 13.

16. Dispositif à plusieurs compartiments ou «kit» de coloration et/ou de décoloration, comprenant un premier compartiment renfermant la composition (A), ainsi qu'un deuxième compartiment renfermant la composition anhydre (B), les compositions (A) et (B) étant telles que définies dans l'une quelconque des revendications 1 à 13.
